# EUROPÄISCHE PATENTSCHRIFT

(11) **EP 0 433 826 B1**
(45) Veröffentlichungstag und Bekanntmachung des Hinweises auf die Patenterteilung: **10.07.1996**
(21) Anmeldenummer: 90123664.6
(22) Anmeldetag: 08.12.1990
(51) Int. Cl.: C07D 213/65, C07D 213/30, C09K 19/34

(54) **Fluorphenylpyridine**
Fluorophenylpyridines
Fluorophénylpyridines

(30) Priorität: 19.12.1989 DE 3941761; 20.06.1990 DE 4019595
(43) Veröffentlichungstag der Anmeldung: 26.06.1991
(73) Patentinhaber: HOECHST AKTIENGESELLSCHAFT, D-65926 Frankfurt am Main (DE)
(72) Erfinder: Wächtler, Andreas, Dr., W-6103 Griesheim (DE); Poetsch, Eike, Dr., W-6109 Mühtal (DE); Geelhaar, Thomas, Dr., W-6500 Mainz (DE); Hittich, Reinhard, Dr., W-6101 Modautal 1 (DE); Reiffenrath, Volker, W-6101 Rossdorf (DE); Kompter, Hans-Michael, Dr., W-6086 Riedstadt-Erfelden (DE)

(56) Entgegenhaltungen:
- EP-A- 0 233 706
- EP-A- 0 242 716
- EP-A- 0 244 939
- EP-A- 0 255 219
- EP-A- 0 257 457
- EP-A- 0 284 093
- EP-A- 0 361 157
- WO-A-86/06373
- WO-A-87/04158
- CHEMICAL ABSTRACTS, Band 110, Nr. 16, 17. April 1989, Seite 684, Zusammenfassung Nr. 145145a, Columbus, Ohio, US; & JP-A-63 253 065 (DAINIPPON INK AND CHEMICALS, INC.) 20-10-1988
- LIQUID CRYSTALS, Band 3, Nr. 12, Dezember 1988, Seiten 1643-1653; M.P. BURROW et al.: "The synthesis and liquid crystal properties of some 2,5-disubstituted pyridines"

## Beschreibung

Die Erfindung betrifft Achirale Fluorphenylpyridine der Formel I4 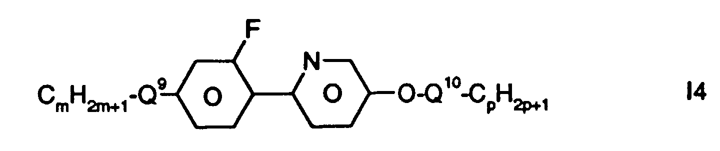 worin
- m und p: jeweils unabhängig voneinander 1 bis 18,
- Q⁹: -O- oder eine Einfachbindung und
- Q¹⁰: -CO-, 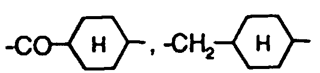 oder eine Einfachbindung bedeutet.

Chirale getiltete smektische flüssigkristalline Phasen mit ferroelektrischen Eigenschaften können hergestellt werden, indem man Basis-Mischungen mit einer oder mehreren getilteten smektischen Phasen mit einem geeigneten chiralen Dotierstoff versetzt (L.A. Beresnev et al., Mol. Cryst. Liq. Cryst. 89, 327 (1982); H.R. Brand et al., J. Physique 44 (lett.), L 771 (1983). Solche Phasen können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367,924) auf der Basis der ferroelektrischen Eigenschaften der chiral getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1-2 µm) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiral getilteten smektischen Phasen (wie z.B. S_{c}*, jedoch auch S_{H}*, S_{I}*, S_{J}*, S_{K}*, S_{G}*, S_{F}*) ist deren geringe chemische, thermische und Photo-Stabilität. Eine weitere nachteilige Eigenschaft von Displays basierend auf derzeit verfügbaren chiral getilteten smektischen Mischungen ist, daß die Spontanpolarisation zu kleine Werte aufweist, so daß das Schaltzeitverhalten der Displays ungünstig beeinflußt wird und/oder der Pitch und/oder der Tilt und/oder die Viskosität der Phasen nicht den Anforderungen der Display-Technologie entspricht. Darüberhinaus ist meist der Temperaturbereich der ferroelektrischen Phasen zu klein und liegt überwiegend bei zu hohen Temperaturen.

Es wurde nun gefunden, daß die Verwendung von Verbindungen der Formel I4 als Komponenten chiral getilteter smektischer Mischungen die erwähnten Nachteile wesentlich vermindern kann. Die Verbindungen der Formel I4 sind somit als Komponenten chiral getilteter smektischer flüssigkristalliner Phasen vorzüglich geeignet. Insbesondere sind mit ihrer Hilfe chemisch besonders stabile chiral getiltete smektische flüssigkristalline Phasen mit günstigen ferroelektrischen Phasenbereichen, günstigen Weiten für die Viskosität, insbesondere mit breiten S_{c}^{*} Phasenbereichen, hervorragender Unterkühlbarkeit bis zu Temperaturen unter 0 °C ohne daß Kristallisation auftritt und für derartige Phasen hohen Werten für die spontane Polarisation herstellbar. P ist die spontane Polarisation in nC/cm². Die Verbindungen der Formel I4 eignen sich jedoch auch für flüssigkristalline Phasen für den elektroklinen Effekt.

Die Verbindungen der Formel I4 weisen eine neutrale Anisotropie der Dielektrizitätskonstanten auf (Δ = -0,2 bis +0,5) und besitzen daher einen breiten Anwendungsbereich. In Abhängigkeit von der Auswahl der Substituenten können diese Verbindungen als Basismaterialien dienen, aus denen flüssigkristalline smektische Phasen zum überwiegenden Teil zusammengesetzt sind; es können aber auch Verbindungen der Formel I4 flüssigkristallinen Basismaterialien aus anderen Verbindungsklassen zugesetzt werden, um beispielsweise die dielektrische und/oder optische Anisotropie und/oder die Viskosität und/oder die spontane Polarisation und/oder den Phasenbereiche und/oder der Tiltwinkel und/oder den Pitch eines solchen Dielektrikums zu variieren.

In der EP 02 52 961 ist eine sehr breite allgemeine Formel für nematische Fluorphenylpyridine angegeben, die teilweise die hier beanspruchten Verbindungen der Formel I4 umfaßt. In der EP 0 252 961 sind keinerlei Hinweise für S_{c}-Verbindungen dieses Typs, vielmehr sollen gerade smektische Phasen unterdrückt werden. Dort sind auch keine Einzelverbindungen der hier beanspruchten Formel genannt.

Chirale Dotierstoffe für ferroelektrische Mischungen werden in den EP-A-0 220 297 und EP-A-0 233 706 beansprucht, deren breite, allgemeine Formeln die erfindungsgemäßen Verbindungen umfassen. In den dort genannten Verbindungen befindet sich jedoch der laterale Halogensubstituent am Phenylring stets in meta-Position zum Pyridinring. Diese weisen jedoch ein positives Δε auf und sind daher für ferroelektrische Mischungen weniger geeignet.

In der EP 0 244 939 werden ähnliche Verbindungen der Formel 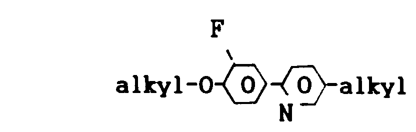 beschrieben, welche jedoch ebenfalls ein positives Δε aufweisen.

In der WO 87-04158 werden ähnliche Verbindungen der Formel 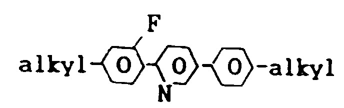 beschrieben.

In der EP 0 361 157 werden 5-Alkyl-2-(4-alkyl-2-fluorphenyl)-pyridine offenbart. M.P. Burow et al., Liquid Crystals, Vol 3, No.12, 1643-1653 beschreibt Derivate des 2-(2-Fluorphenyl)-pyridins mit drei Ringen der Formel

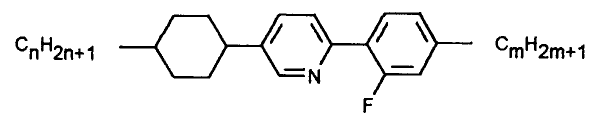

In der japanischen Offenlegungsschrift JP 63,253,065 werden Verbindungen der Formel 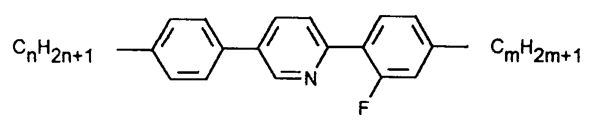 offenbart.

Chirale Derivate des 2-(2-Fluorphenyl)-pyridins werden zum Beispiel von der EP 0 284 093, der EPO 242 716, der EP 0 255 219 und der EP 0 257 457 umfaßt.

Der Fachmann konnte somit aus dem Stand der Technik weder in einfacher Art und Weise Synthesemöglichkeiten für die beanspruchten Verbindungen entnehmen noch erkennen, daß die erfindungsgemäßen Verbindungen überwiegend breite und günstig gelegene S_{c}-Phasen aufweisen sowie sich durch günstige Werte für die Rotationsviskosität auszeichnen.

Gegenstand der Erfindung sind somit die Fluorphenylpyridine der Formel I4.

Gegenstand der Erfindung sind ferner ferroelektrische flüssigkristalline Phasen mit einem Gehalt an mindestens einer Verbindung der Formel I4 sowie Flüssigkristallanzeigelemente, insbesondere ferroelektrische elektrooptische Anzeigeelemente, die derartige Phasen enthalten.

Die erfindungsgemäßen Phasen enthalten vorzugsweise mindestens zwei, insbesondere mindestens drei Verbindungen der Formel I4. Besonders bevorzugt sind erfindungsgemäße chirale getiltete smektische flüssigkristalline Phasen, deren achirale Basismischung neben Verbindungen der Formel I4 mindestens eine andere Komponente mit negativer oder betragsmäßig kleiner positiver dielektrischer Anisotropie enthält. Diese weiteren Komponente(n) der achiralen Basismischung können 1 bis 50 %, vorzugsweise 10 bis 25 %, der Basismischung ausmachen. Als weitere Komponenten mit betragsmäßig kleiner positiver oder negativer dielektrischer Anisotropie eignen sich Verbindungen der Formel IV, welche die Verbindungen der Teilformeln IVa bis IVi umfaßt: 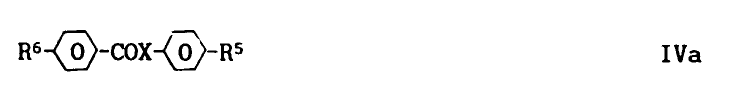 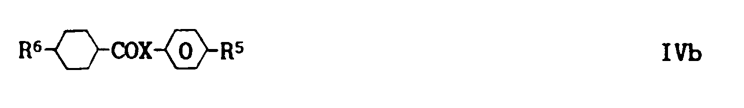 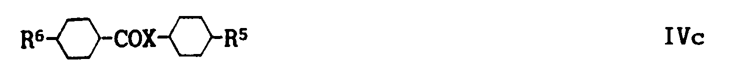 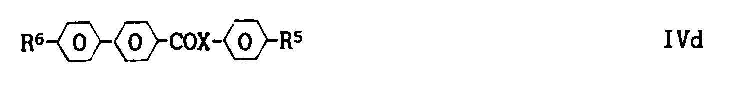 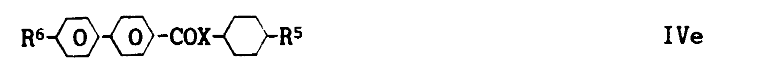 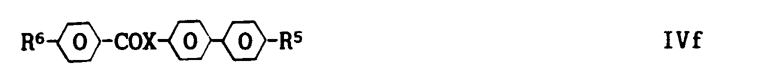 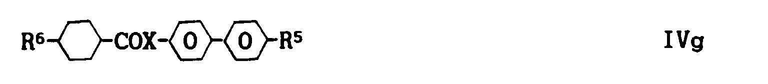 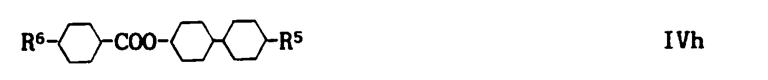 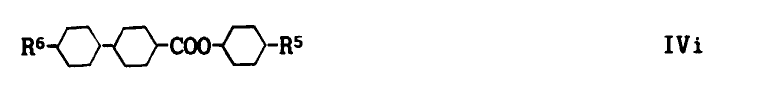 R⁶ und R⁵ sind jeweils Alkyl mit 1 bis 15 C-Atomen worin auch eine CH₂-Gruppe durch -O-, -CO-O- oder -O-CO- ersetzt sein kann, vorzugsweise geradkettiges Alkyl, Alkoxy, Alkanoyloxy oder Alkoxycarbonyl mit jeweils 3 bis 12 C-Atomen. X ist S oder O, vorzugsweise O. In den Verbindungen der Formeln IVa, IVb, IVd, IVe, IVf und IVg kann auch eine 1,4-Phenylengruppe lateral durch Halogen oder CN, insbesondere bevorzugt durch Fluor, substituiert sein.

Besonders bevorzugt sind die Verbindungen der Teilformeln IVa, IVb, IVd und IVf, worin R⁶ und R⁵ jeweils geradkettiges Alkyl oder Alkoxy mit jeweils 5 bis 10 C-Atomen bedeutet.

Besonders bevorzugte Einzelverbindungen sind in der folgenden Tabelle I angegeben:

**Tabelle I**

| | | | |
|---|---|---|---|
| Formel | R⁶ | R⁵ | X |
| IVa | n-Decyloxy | n-Heptyloxy | O |
| IVa | n-Hexyloxy | n-Decyloxy | O |
| IVa | n-Octyloxy | n-Heptyl | O |
| IVa | n-Octyloxy | n-Pentyl | O |
| IVa | n-Decyloxy | n-Heptyl | O |
| IVa | n-Decyloxy | n-Pentyl | O |
| IVf | n-Pentyl | n-Pentyl | O |
| IVf | n-Pentyl | n-Hexyl | O |

Die Verbindungen der Teilformeln IVc, IVh und IVi eignen sich als Zusätze zur Schmelzpunktserniedrigung und werden normalerweise den Basismischungen mit nicht mehr als 5%, vorzugsweise 1 bis 3 %, zugesetzt. R⁶ und R⁵ bedeuten in den Verbindungen der Teilformeln IVc, IVh und IVi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel 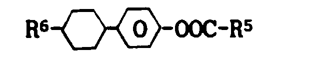 worin R⁶ und R⁵ die für IVc, IVh und IVi angegebene bevorzugte Bedeutung haben.

Als weitere Komponenten mit negativer dielektrischer Anisotropie eignen sich weiterhin Verbindungen enthaltend das Strukturelement A, B oder C.

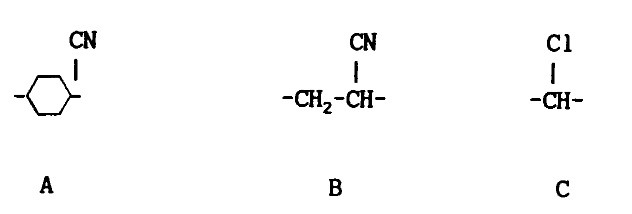

Bevorzugte Verbindungen dieser Art entsprechen den Formeln Va, Vb und Vc:

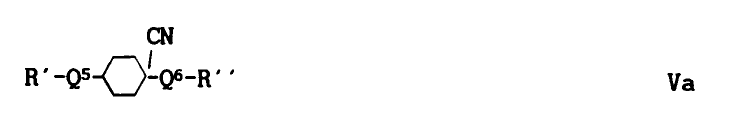

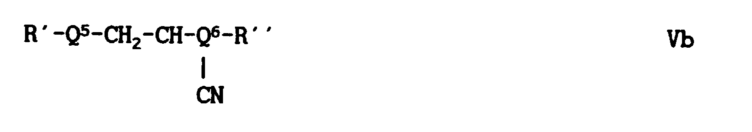

R'-Q⁷-Q⁸-R''' VC

R' und R'' bedeuten jeweils Alkyl mit 1 bis 15 C-Atomen, worin auch eine CH₂-Gruppe durch -O- ersetzt sein kann, vorzugsweise geradkettige Alkyl- oder Alkoxy-Gruppen mit jeweils 2 bis 10 C-Atomen. Q⁵ und Q⁶ bedeuten jeweils 1,4-Phenylen, trans-1,4-Cyclohexylen, 4,4'-Biphenylyl, 4-(trans-4-Cyclohexyl)-phenyl, trans,trans-4,4'-Bicyclohexyl oder eine der Gruppen Q⁵ und Q⁶ auch eine Einfachbindung.

Q₇ und Q₈ bedeuten jeweils 1,4-Phenylen, 4,4'-Biphenylyl oder trans-1,4-Cyclohexylen. Eine der Gruppen Q⁷ und Q⁸ kann auch 1,4-Phenylen bedeuten, worin mindestens eine CH-Gruppe durch N ersetzt ist. R''' ist ein optisch aktiver Rest, vorzugsweise ein n-Alkyl- oder Alkoxyrest mit 3 bis 12 C-Atomen mit einem asymmetrischen Kohlenstoffatom der Struktur 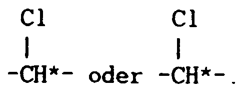 Besonders bevorzugte Verbindungen der Formel Vc sind diejenigen der Formel Ic': 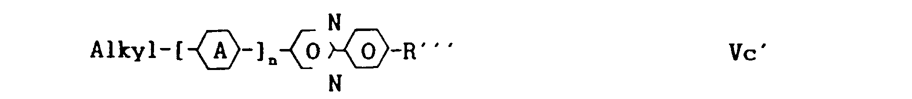 worin A 1,4-Phenylen oder trans-1,4-Cyclohexylen und n 0 oder 1 bedeutet.

Die Verbindungen der Formel I4 umfassen die nachstehend angeführten nicht-chiralen bevorzugten zweikernigen und dreikernigen Materialien der Formeln Ib und Ie, worin m und p jeweils unabhängig 1 bis 18 bedeuten. 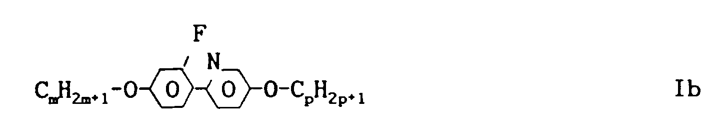 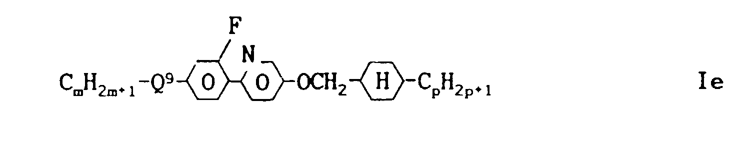 m ist vorzugsweise 5 bis 14, insbesondere 6 bis 12. p ist vorzugsweise 3 bis 12. Die Reste CₘH₂ₘ₊₁ und CₚH₂ₚ₊₁ sind vorzugsweise geradkettig. Verbindungen der Formel I4 mit relativ kurzen derartigen Resten eignen sich auch als Komponenten nematischer Phasen.

Die Verbindungen der Formel I4 werden nach an sich bekannten Methoden, wie sie in der Literatur (z.B. in den Standardwerken wie Houben-Weyl, Methoden der Organischen Chemie, Georg-Thieme-Verlag, Stuttgart) beschrieben sind, hergestellt.

Die Ausgangsstoffe können gewünschtenfalls auch in situ gebildet werden, derart, daß man sie aus dem Reaktionsgemisch nicht isoliert, sondern sofort weiter zu den Verbindungen der Formel I4 umsetzt.

Die erfindungsgemäßen Verbindungen lassen sich nach folgenden Reaktionsschemata (Schema 1 bis Schema 3) einfach herstellen (R¹ = CₘH₂ₘ₊₁-Q⁹-, R² = -O-Q¹⁰-CₚH₂ₚ₊₁).

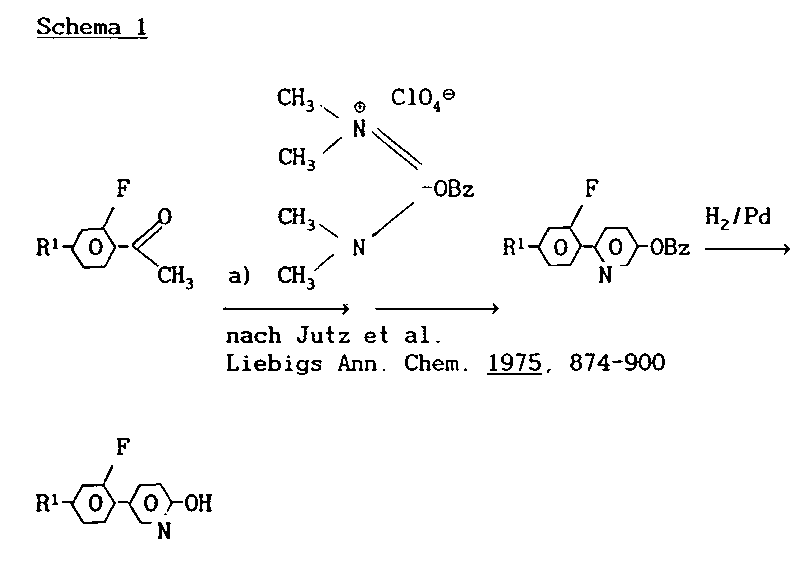

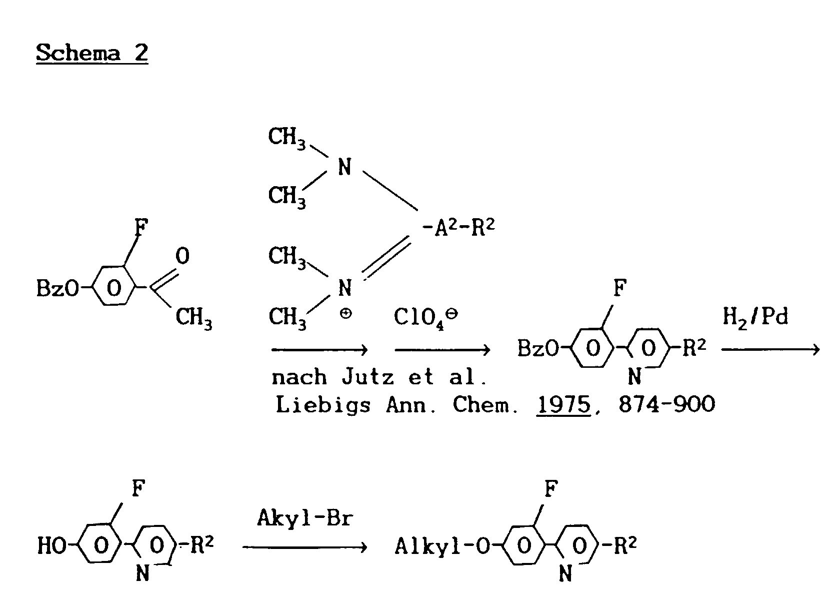

Die erfindungsgemäßen Verbindungen sind weiterhin durch Kopplung von metallorganischen Zinkverbindungen mit entsprechenden Brompyridinderivaten entsprechend DE-OS 36 32 410 erhältlich.

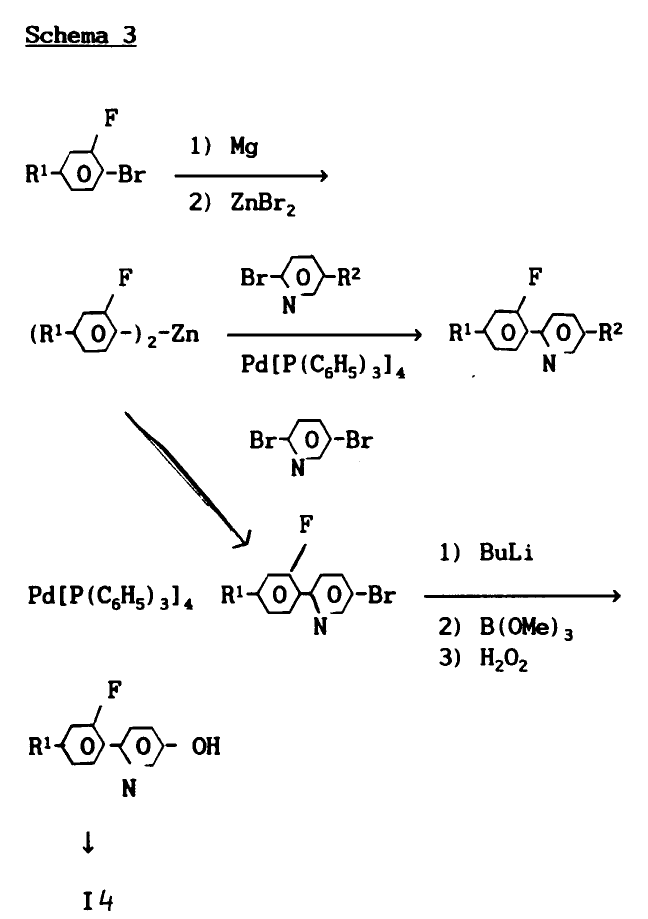

Die zur Kreuz-Kopplung nach Schema 1 notwendige Boronsäure erhält man auch durch direkte ortho-Metallierung nach

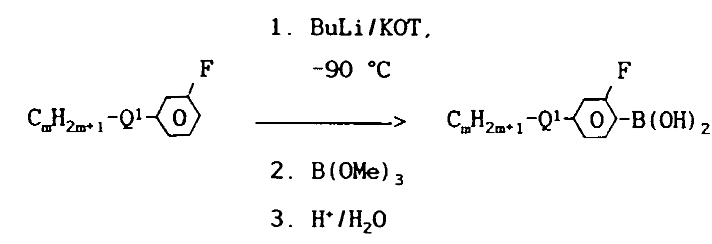

Im folgenden wird die Synthese einiger besonders interessanter Hydroxy-Zwischenstufen beschrieben:
a) 5-Hydroxy-2-(2-fluor-4-alkylphenyl)pyridine bzw. 5-Hydroxy-2-(2-fluor-4-alkoxyphenyl)pyridine sind erhältlich aus 2-Benzyloxytrimethiniumsalz durch Kondensation mit 4-Alkyl- oder 4-Alkoxy-2-fluoracetophenonen, Umsetzung mit NH₃/NH₄Cl oder Ammoniumacetat,
   analog den Vorschriften von Ch. Jutz et al. (Liebigs Ann. Chem. 1975 874-900) und anschließende Hydrogenolyse oder aus 4-Alkyl- bzw. 4-Alkoxy-2-fluorphenylboronsäure durch Kopplung mit 5-Acetoxy-2-brompyridin (erhältlich aus 5-Hydroxy-2-brompyridin durch Veresterung) in Gegenwart eines Pd-Katalysators entsprechend den Arbeiten von Suzuki et al. (Synth. Commun. 11 513-19 (1981)).
b) 5-Alkoxy-2-(2-fluor-4-hydroxyphenyl)pyridine sind erhältlich durch Kopplung von 4-Benzyloxy-2-fluorphenylboronsäure mit 5-Alkoxy-2-brompyridin entsprechend oben genannter Literatur und anschließende Hydrogenolyse.

Eine weitere prinzipielle Möglichkeit zur Herstellung von 2(subst-Phenyl)-5-alkoxypyridinen ergibt sich durch den Cu-katalysierten Brom-Alkoxyaustausch bei den entsprechenden 2(subst-Phenyl)-5-brompyridinen (zugänglich durch Kreuzkopplung von 2,5-Dibrompyridin mit entsprechend substituierter Phenylboronsäure unter den Bedingungen von Snieckus) analog der am Beispiel des Brombenzols durchgeführten Arbeiten von H.L. Aalten (Tetrahedron 45 (17) 5565 (1989)) sowie ein Brom-Alkoxyaustausch bei den N-Oxiden der entsprechenden 5-Brom-Pyridinen. Die auf dem zuletzt genannten Weg erhaltene 5-Alkoxy-Pyridin-N-oxide müssen anschließend nach literaturbekannter Methode zu 5-Alkoxy-Pyridin reduziert werden.

Diese erfindungsgemäßen flüssigkristallinen Phasen bestehen aus 2 bis 25, vorzugsweise 3 bis 15 Komponenten, darunter mindestens einer Verbindung der Formel I4. Die anderen Bestandteile werden vorzugsweise ausgewählt aus den nematischen oder nematogenen Substanzen, insbesondere den bekannten Substanzen, aus den Klassen der Azorybenzole, Benzylidenaniline, Biphenyle, Terphenyle, Phenyl-oder Cyclohexylbenzoate, Cyclohexancarbonsäurephenyl- oder cyclohexylester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexylpyridazine sowie deren N-Oxide, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel I' charakterisieren,

R'-L-G-E-R" I'

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH- | -N(O)=N- |
| | -CH=CY- | -CH=N(O)- |
| | -C=C- | -CH₂-CH₂- |
| | -CO-O- | -CH₂-O- |
| | -CO-S- | -CH₂-S- |
| | -CH=N- | -COO-Phe-COO- |

oder eine C-C-Einfachbindung,
Y Halogen, vorzugsweise Chlor, oder -CN, und
R' und R'' Alkyl, Alkoxy, Alkanoyloxy, Alkoxycarbonyl oder Alkoxycarbonyloxy mit bis zu 18, vorzugsweise bis zu 8 Kohlenstoffatomen, oder einer dieser Reste auch CN, NC, NO₂, CF₃, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99 %, vorzugsweise 10 bis 95 %, einer oder mehrerer Verbindungen der Formel I4. Weiterhin bevorzugt sind erfindungsgemäße flüssigkristalline Phasen, enthaltend 0,1-40 %, vorzugsweise 0,5-30 % einer oder mehrerer Verbindungen der Formel I4.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol. Cryst.Liq.Cryst. Band 24, Seiten 249-258 (1973)) zur Verbesserung der Leitfähigkeit, pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vorund nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Weiterhin bedeuten:
- K: kristallin
- N: nematisch
- S: smektisch
- I: isotrop

Die zwischen diesen Symbolen stehenden Zahlen geben die jeweilige Phasenübergangstemperatur in °C an. Ferner werden folgende Abkürzungen verwendet:
- DAST: Diethylaminoschwefeltrifluorid
- DMF: Dimethylformamid
- n-BuLi: n-Butyllithium

### Beispiel 1

0,1 mol 2-(4-Nonyl-2-fluorphenyl)-5-hydroxypyridin (hergestellt durch Kondensation von 4-Nonyl-2-fluor-acetophenon mit 2-Benzyloxytrimethiniumperchlorat nach Jutz et al. (siehe Seite 17)) mit anschließender Hydrogenolyse des Benzylethers) werden mit 0,11 mol 1-Bromheptan und 0,11 mol Kaliumcarbonat in Dimethylformamid als Lösungsmittel verethert. Nach der Aufarbeitung wird das 2-(4-Nonyl-2-fluorphenyl)-5-heptyloxypyridin aus Isopropanol umkristallisiert.

Analog erhält man durch Umsetzung von 4-Benzyloxy-2-fluoracetophenon mit 2-Octyloxytrimethiniumperchlorat, Ammonium acetat und hydrierende Spaltung des Benzylethers 2-(4-Hydroxy-2-fluorphenyl)-5-octyloxypyridin, das mit 1-Bromdecan zum 2-(4-n-Decyloxy-2-fluorphenyl)-5-octyloxypyridin alkyliert wird.

Analog werden folgende Verbindungen der Formel I4 hergestellt:

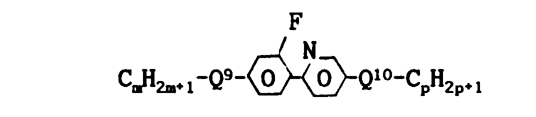

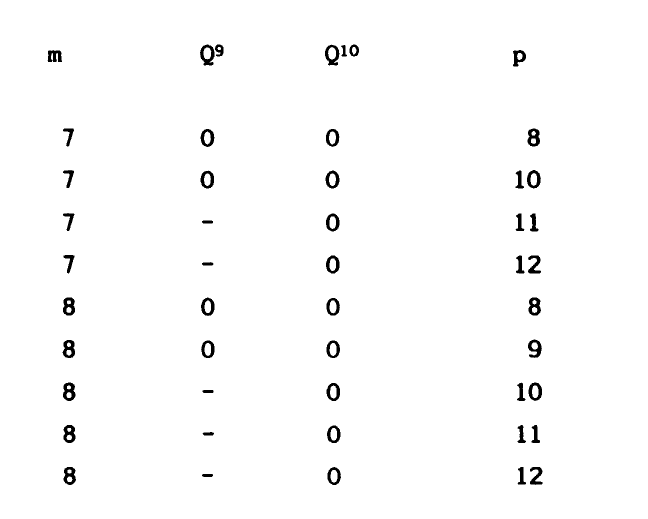

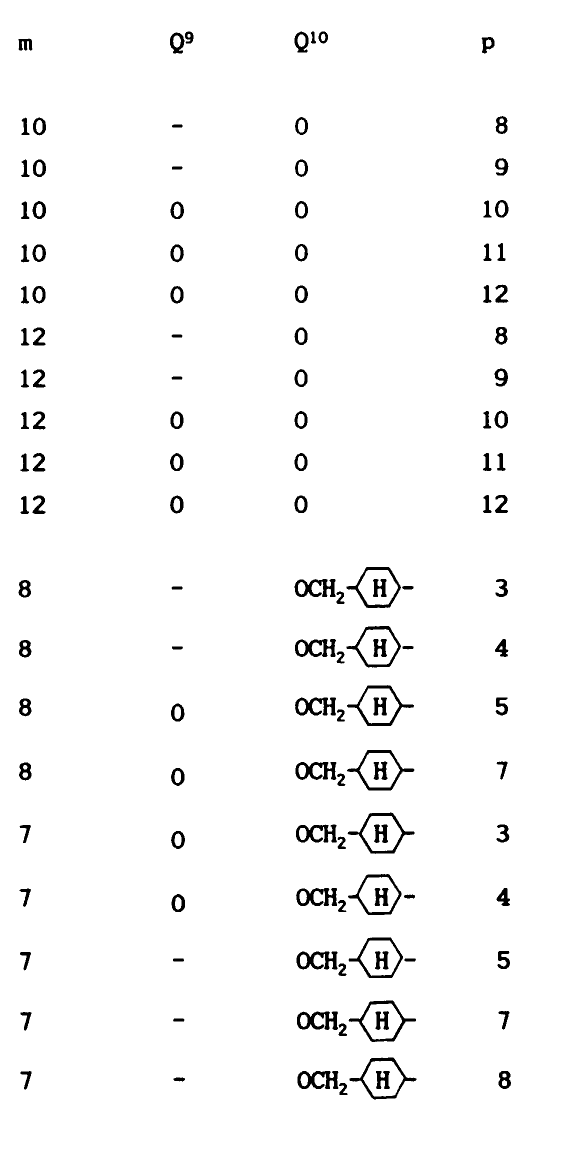

### Beispiel 2

0,1 mol 2-(4-Heptyloxy-2-fluorphenyl)-5-hydroxypyridin (hergestellt aus 4-Heptyloxy-2-fluoracetophenon entsprechend Beispiel 1) wird zusammen mit 0,12 mol Pyridin in Toluol gelöst und dann bei Raumtemperatur 0,1 mol Nonansäurechlorid zugegeben. Dann wird 12 Stunden gerührt und wie üblich aufgearbeitet. Man erhält 2-(4-Heptyloxy-2-fluorphenyl)-5-nonanoyloxy-pyridin.

Analog erhält man folgende Ester der Formel I4:

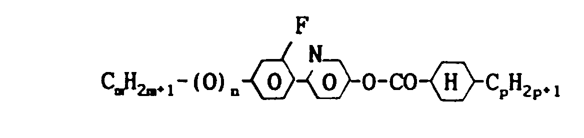

| m | n | p |
|---|---|---|
| 8 | 1 | 3 |
| 8 | 1 | 5 |
| 8 | 1 | 7 |
| 8 | 1 | 9 |
| 8 | 1 | 10 |
| 8 | 1 | 11 |
| 8 | 0 | 3 |
| 8 | 1 | 5 |
| 10 | 0 | 3 |
| 7 | 1 | 7 |
| 7 | 1 | 11 |
| 7 | 0 | 3 |

### Referenz-Beispiel

### Herstellung von 2-(4-Octyloxy-2-fluorphenyl)-5-methylpyridin

0,04 mol 2-Fluor-4-octyloxyphenylboronsäure, 0,035 mol 2-Brom-5-methylpyridin und 1,4 g Pd(PPh₃)₄ als Katalysator werden in einem Lösungsmittelgemisch aus 65 ml Toluol, 25 ml Ethanol und 40 ml einer 2 m Na₂CO₃-Lösung unter Ausschluß von Luftsauerstoff 18 Stunden am Rückfluß gekocht. Dann wird wie üblich aufgearbeitet und das Produkt chromatographisch und durch Kristallisation gereinigt.

### Herstellung von 2-(4-Octyloxy-2-fluorphenyl)-5-nonylpyridin

Unter Ausschluß von Luftsauerstoff und Feuchtigkeit gibt man bei -70 °C zu einer Lösung aus 8,7 mmol LDA (hergestellt aus BuLi und Diisopropylamin) in THF in Gegenwart von 1,04 ml DMPU 7,9 mmol 2-(4-Octyloxy-2-fluorphenyl)-5-methylpyridin gelöst in THF und nach etwa 15 Minuten 8,7 mmol 1-Bromoctan. Man läßt das Reaktionsgemisch sich auf Raumtemperatur erwärmen, rührt noch eine weitere Stunde und arbeitet wie üblich auf.

### Beispiele 3 bis 44:

Analog bzw. nach obigem Schemata und ggf. nachfolgender Veretherung oder Veresterung entsprechender Hydroxyverbindungen nach Standardmethoden werden folgende Verbindungen der Formel I4 hergestellt:

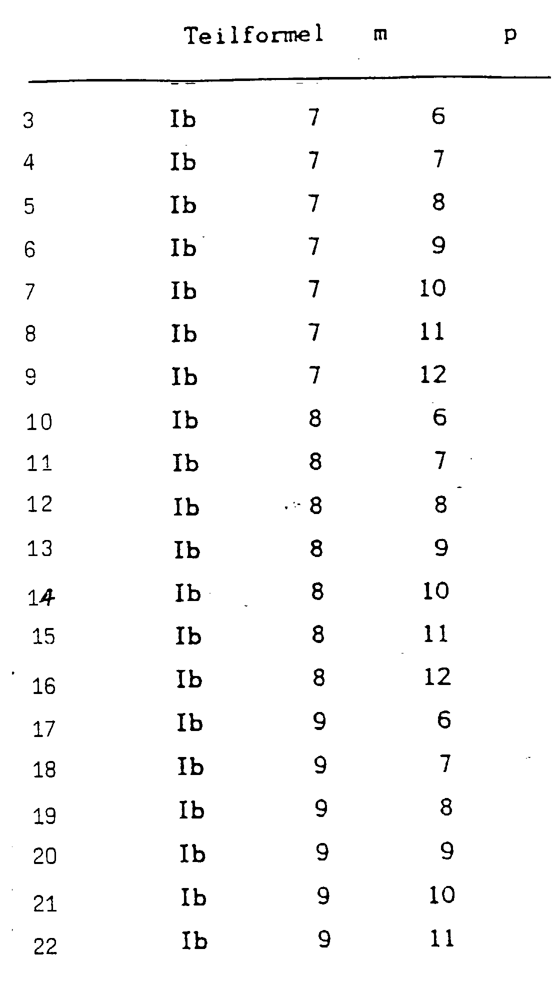

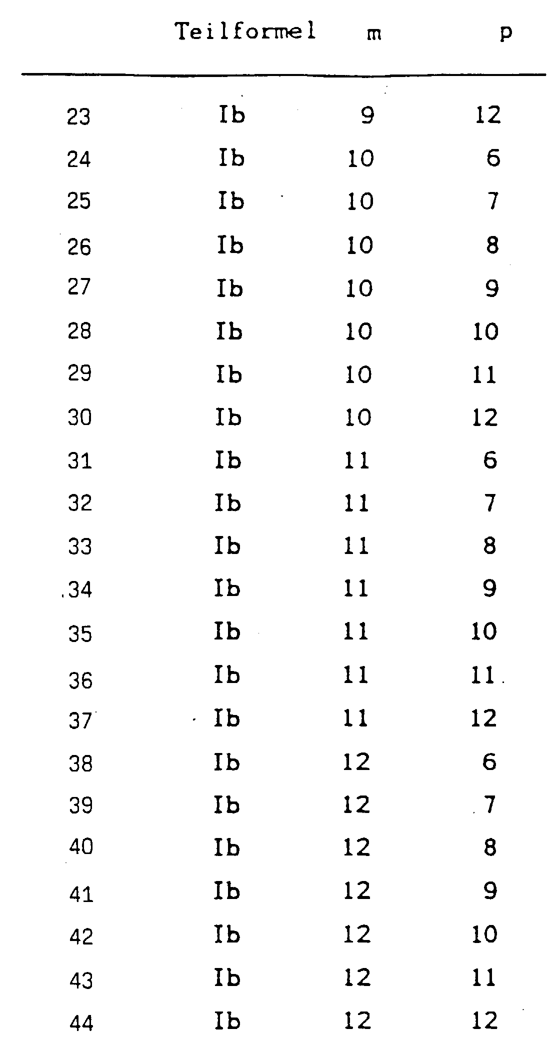

Die folgenden Beispiele betreffen ferroelektrische flüssigkristalline Medien.

### Beispiel A

Man stellt ein flüssigkristallines Medium her bestehend aus:

| | |
|---|---|
| 5,8 % | (2-Hexyloxyphenyl)-5-heptylpyrimidin |
| 5,8 % | (2-Octyloxyphenyl)-5-heptylpyrimidin |
| 5,8 % | (2-Decyloxyphenyl)-5-heptylpyrimidin |
| 1,1 % | (2-p-Octyloxyphenyl)-5-octylpyrimidin |
| 1,1 % | (2-p-Nonyloxyphenyl)-5-octylpyrimidin |
| 1,1 % | (2-p-Decyloxyphenyl)-5-octylpyrimidin |
| 7,0 % | 2-(4-Decyloxy-2-fluorphenyl)-5-docecyloxypyridin |
| 14,1 % | 2-(4-Heptyloxy-2-fluorphenyl)-5-dodecyloxypyridin |
| 14,1 % | 2-(4-Decyloxy-2-fluorphenyl)-5-undecyloxypyridin |
| 5,0 % | 2-(4-Octyloxy-2,3-difluorphenyl)-5-nonylpyrimidin |
| 5,0 % | 2-(4-Heptyloxy-2,3-difluorphenyl)-5-nonylpyrimidin |
| 5,0 % | 2-(4-Nonyloxy-2,3-difluorphenyl)-5-nonylpyrimidin |
| 18,0 % | 2-(4-Hexyloxyphenyl)-5-hexyloxypyrimidin |
| und | |
| 10 % | optisch aktivem |
| | 2-[4-(2-Fluoroctyloxy)-2,3-difluorphenyl]-5-heptylpyrimidin. |

Dieses Medium weist einen breiten S_{c}*-Bereich auf:

### Beispiel B

Man stellt ein flüssigkristallines Medium her bestehend aus:

| | |
|---|---|
| 5,8 % | 2-(p-Hexyloxyphenyl)-5-heptylpyrimidin |
| 5,8 % | 2-(p-Octyloxyphenyl)-5-heptylpyrimidin |
| 5,8 % | 2-(p-Decyloxyphenyl)-5-heptylpyrimidin |
| 1,1 % | 2-(p-Octyloxyphenyl)-5-octylpyrimidin |
| 1,1 % | 2-(p-Nonyloxyphenyl)-5-octylpyrimidin |
| 2,2 % | 2-(p-Decyloxyphenyl)-5-octylpyrimidin |
| 5,0 % | 2-(4-Decyloxy-2-fluorphenyl)-5-dodecyloxypyridin |
| 10,1 % | 2-(4-Heptyloxy-2-fluorphenyl)-5-dodecyloxypyridin |
| 10,1 % | 2-(4-Decyloxy-2-fluorphenyl)-5-undecyloxypyridin |
| 10 % | 2-(4-Octyloxy-2,3-difluorphenyl)-5-heptylpyrimidin |
| 5 % | 2-(4-Octyloxy-2,3-difluorphenyl)-5-nonylpyrimidin |
| 5 % | 2-(4-Heptyloxy-2,3-difluorphenyl)-5-nonylpyrimidin |
| 5 % | 2-(4-Nonyloxy-2,3-difluorphenyl)-5-nonylpyrimidin |
| 18 % | 2-(p-Hexyloxyphenyl)-5-hexyloxypyrimidin |
| und | |
| 10 % | optisch aktivem 2-[4-(2-Fluoroctyloxy)-2-fluorphenyl]-5-heptyloxypyridin |

Dieses Medium weist einen S_{c}*-Phasenbereich von über 60 °C und eine hohe Spontanpolarisation auf.

### Beispiel C

Man stellt ein flüssigkristallines Medium her bestehend aus:

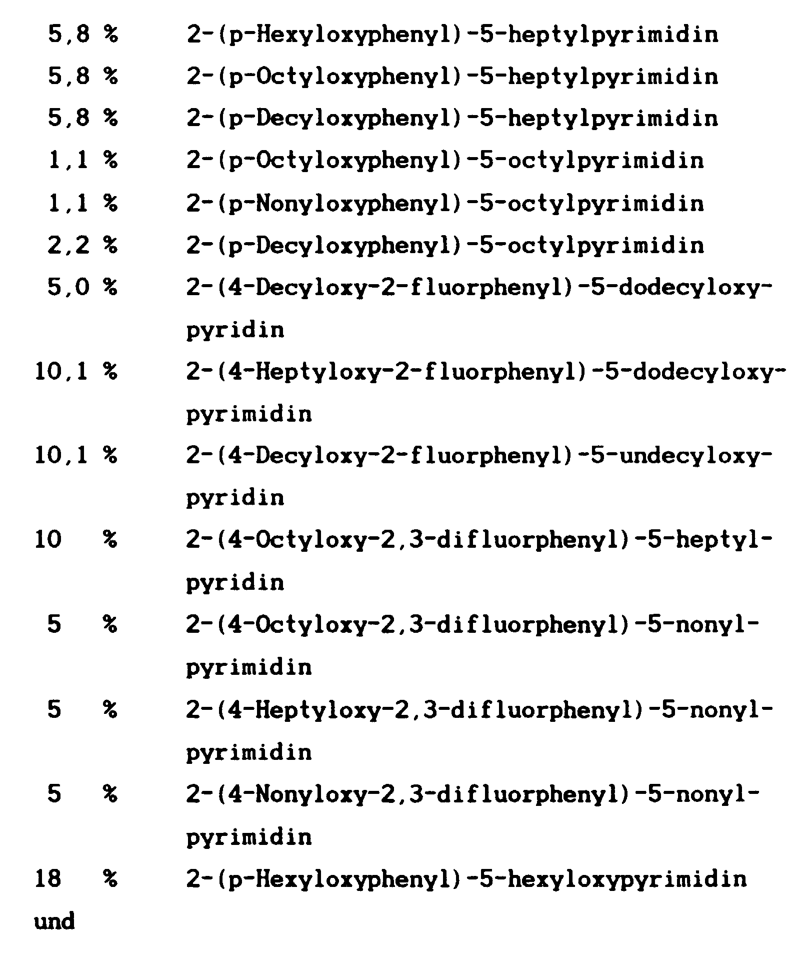

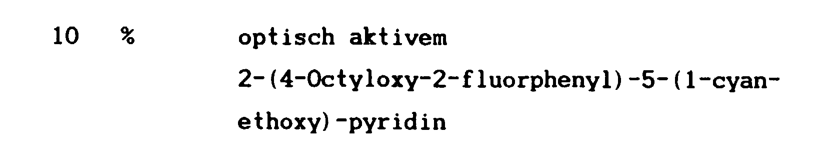

Dieses Medium weist einen S_{c}*-Phasenbereich von über 50 °C und eine hohe Spontanpolarisation auf.

### Beispiel D

Man stellt ein flüssigkristallines Medium her bestehend aus:

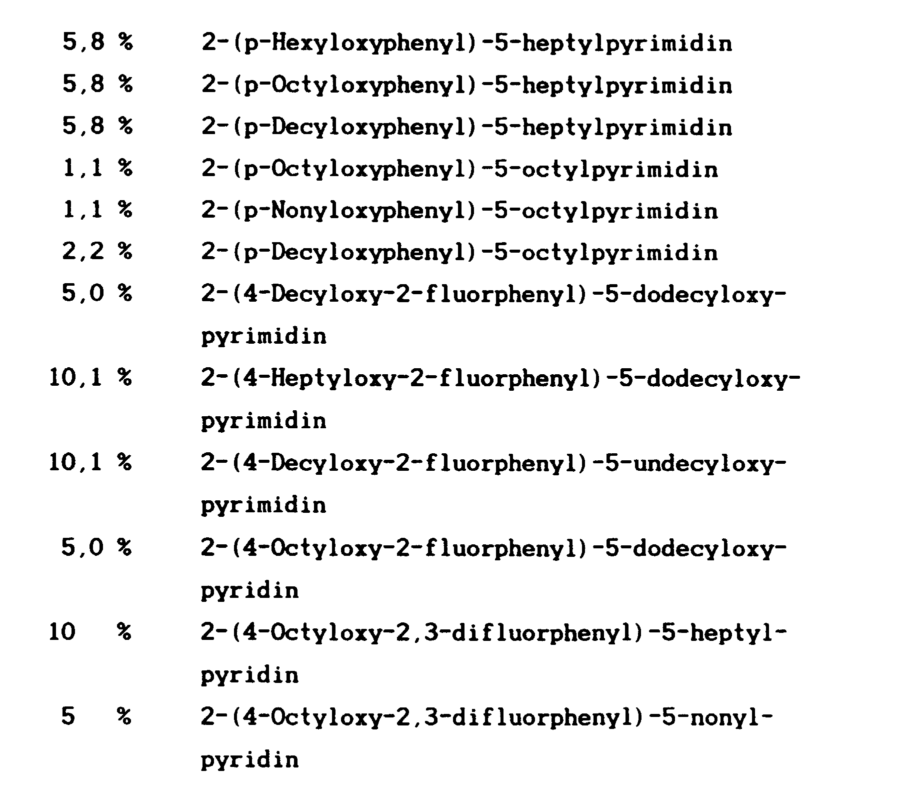

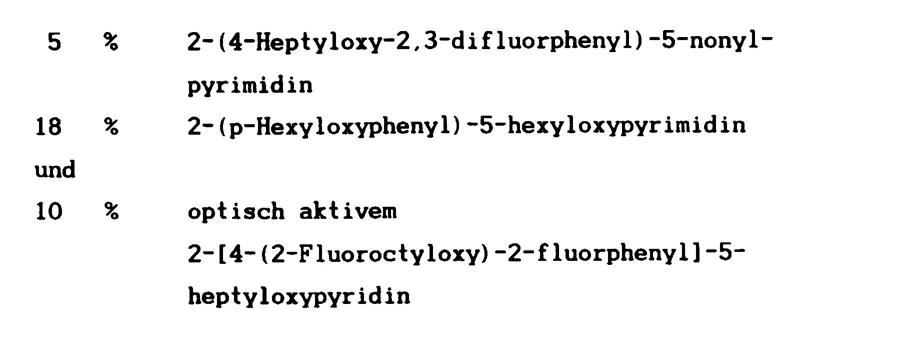

Dieses Medium weist einen breiten S_{c}*-Phasenbereich und eine hohe Spontanpolarisation auf.

### Beispiel E

Man stellt ein flüssigkristallines Medium her bestehend aus:

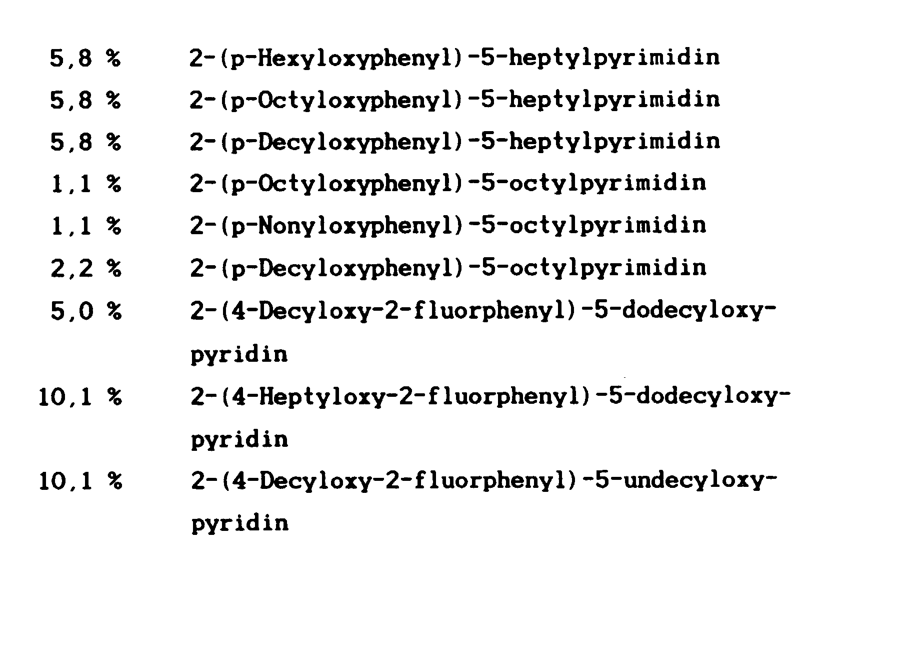

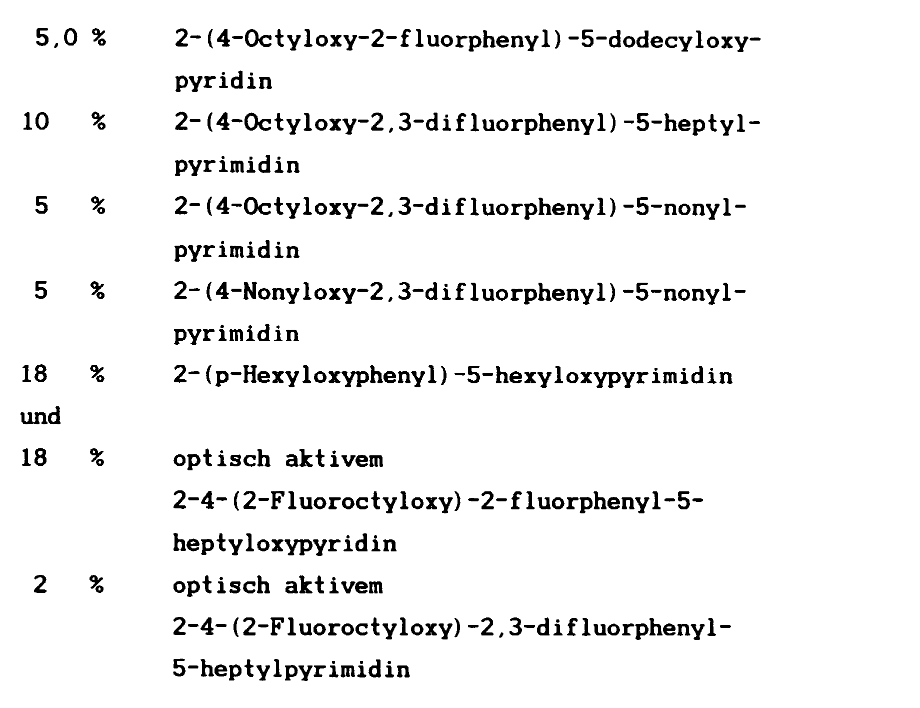

Dieses Medium weist einen breiten S_{c}*-Phasenbereich und eine hohe Spontanpolarisation auf.

## Patentansprüche

1. Achirale Fluorphenylpyridine der Formel I4 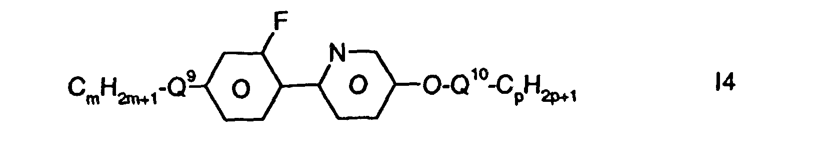 worin
m und p jeweils unabhängig voneinander 1 bis 18,
Q⁹ -O- oder eine Einfachbindung und
Q¹⁰ -CO-, 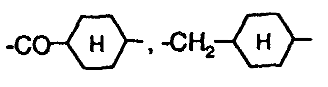 oder eine Einfachbindung bedeutet.

2. Fluorphenylpyridine nach Anspruch 1 ausgewählt aus den Formeln Ib und Ie 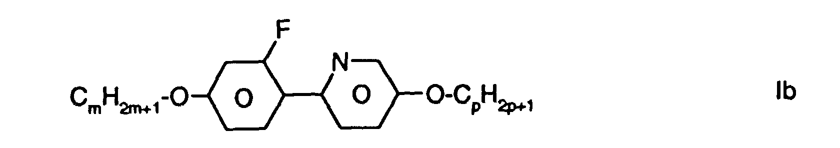 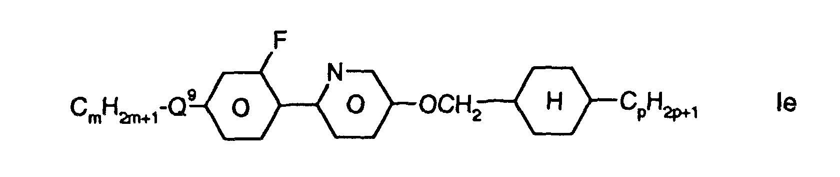 worin
Q⁹, m und p die angegebene Bedeutung besitzen.

3. Flüssigkristallines Medium mit mindestens zwei flüssigkristallinen Komponenten, dadurch gekennzeichnet, daß mindestens eine Komponente eine Verbindung der Formel 14 gemäß Anspruch 1 ist.

4. Flüssigkristallanzeigeelement, dadurch gekennzeichnet, daß es ein flüssigkristallines Medium nach Anspruch 3 enthält.

5. Elektrooptisches Anzeigeelement, dadurch gekennzeichnet, daß es als Dielektrikum ein flüssigkristallines Medium nach Anspruch 3 enthält.

## Claims

1. An achiral fluorophenylpyridine of the formula I4 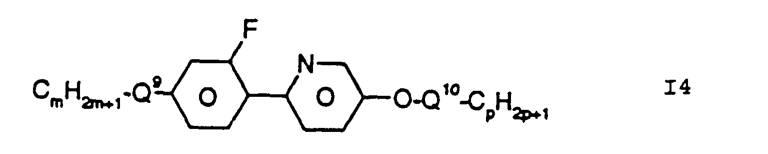 where
m and p are, in each case independently of each other, 1 to 18,
Q⁹ is -O- or a single bond, and
Q¹⁰ is -CO-, 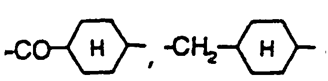 or a single bond.

2. A fluorophenylpyridine as claimed in claim 1 selected from the formulae Ib and Ie 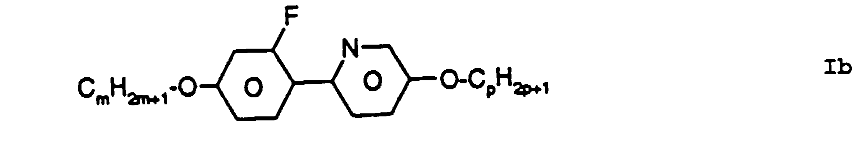 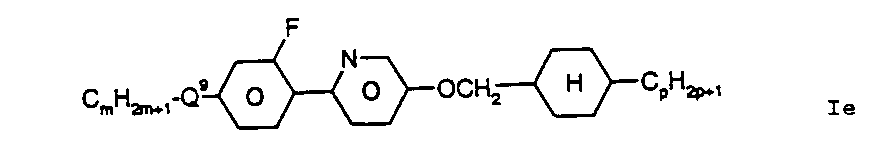 where
Q⁹, m and p have the specified meaning.

3. A liquid-crystalline medium containing at least two liquid-crystalline components, wherein at least one component is a compound of the formula I4 as claimed in claim 1.

4. A liquid-crystal display element which contains a liquid-crystalline medium as claimed in claim 3.

5. An electro-optical display element which contains a liquid-crystalline medium as claimed in claim 3 as dielectric.

## Revendications

1. Fluorophénylpyridines achirales de formule I4 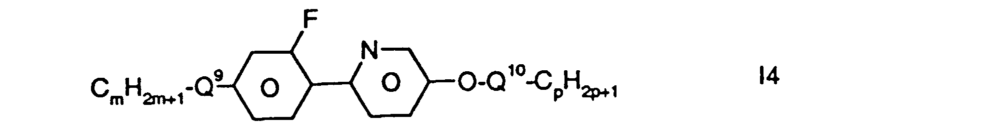 dans laquelle
m et p, indépendamment l'un de l'autre, vont de 1 à 18
Q⁹ représente -O- ou une liaison simple et
Q¹⁰ représente -CO-, 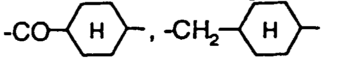 ou une simple liaison.

2. Fluorophénylpyridines selon la revendication 1, pris parmi les formules Ib et Ie 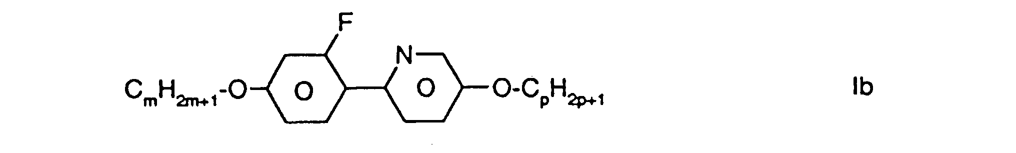 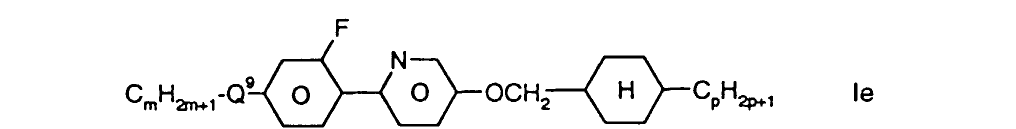 où
Q⁹, m et p ont les signification données.

3. Milieu à cristaux liquides avec au moins deux composants à cristaux liquides, caractérisé en ce que au moins un composant répond à la formule I4 selon la revendication 1.

4. Elément d'affichage à cristaux liquides caractérisé en ce qu'il contient un milieu à cristaux liquides selon la revendication 3.

5. Elément d'affichage électro-optique, caractérisé en ce qu'il contient comme diélectrique un milieu à cristaux liquides selon la revendication 3.
